# EUROPEAN PATENT APPLICATION

(11) **EP 1 808 697 A1**
(43) Date of publication of application: **18.07.2007**
(21) Application number: 06000816.6
(22) Date of filing: 13.01.2006
(51) Int. Cl.: G01N 33/569, C12N 7/02

(54) **Use of an ion exchange matrix for determining the concentration of virus particles and/or virus antigens**

(71) Applicant: Novartis Vaccines and Diagnostics, Inc., Emeryville, CA 94608 (US)
(72) Inventor: Kost, Holger, Dr., 35713 Eschenburg (DE)
(74) Representative: UEXKÜLL & STOLBERG

(57) **Abstract**

The invention provides a method for determining the concentration of virus particles and/or virus antigens in a sample. In particular, the invention relates to determining the concentration of influenza virus particles/influenza virus antigens in a sample. The invention further relates to the use of an ion-exchange matrix for the determination of the concentration of virus particles and/or virus antigens in a sample.

## Description

The invention provides a method for determining the concentration of virus particles/virus antigens in a sample. In particular, the invention relates to determining the concentration of influenza virus particles/influenza virus antigens in a sample. The invention further relates to the use of an ion-exchange matrix for the determination of the concentration of virus particles/virus antigens in a sample.

### Background of the invention

The preparation of vaccines conferring protection against virus-related diseases regularly requires large quantities of virus. Consequently, the respective viruses have to be produced in high yields in a suitable host system. Several methods for the propagation and culturing of viruses have been described in the prior art. Common methods of virus propagating include, for example, culturing the virus in primary cells or in even more complex cell culture systems. For example, influenza vaccines can be derived from virus propagation cultures which make use of embryonate chicken eggs. In these cultures, the virus is isolated from the allantoic fluid of infected eggs and the antigens are used as a vaccine either in the form of complete or disintegrated virus particles. Several viruses, such as influenza, rabies, mumps, measles, rubella and tick-borne encephalitis virus may also be replicated by use of specific mammalian cell lines. These cell cultures allow for economical virus replication and provide a more reliable propagation system than, for example, chicken eggs. Methods for the culturing of viruses in mammalian cells are disclosed, for example in WO 97/37000.

In order to achieve a high virus yield, the host system, culturing conditions and the virus to be propagated must be adapted to one another. Although efforts have been made to optimize distinct propagation systems, the primary virus yield of a virus propagation culture can considerably vary between different batches or production runs. Thus, it is important to provide methods by use of which the virus yield of a certain virus preparation can be determined.

Currently, the determination of primary yield of an influenza virus propagation culture is performed, for example, by measuring the haemagglutinin (HA) titer. Here, the aggregation of hen erythrocytes is used to indicate the amount of virus-derived haemagglutinin in the cell-free virus preparations, such as a crude sample aliquot of a given culture. By using increasing dilutions of such samples, an assessment of the HA amount can be made. The amount of haemagglutinin is indicative for the number of viral particles in the sample. However, this method is strain-specific and, moreover, only semiquantitative. In fact, only factor 2-steps in sensitivity can be determined.

Alternatively, influenza virus yield can be assessed by employing the well-accepted single radial immunodiffusion (also referred to in literature as SRID or SRD test). This test is described in detail in the European Pharmacopoeia, 5^{th} edition. The test is based on the visible reaction of a virus antigen and its homologeous antibody in a supporting agarose gel. It is performed by incorporating the virus antigen into the gel and allowing antisera to diffuse radially from the points of application through the fixed antigens. Measurable zones resulting from the antigen-antibody complexes can be observed. When the equilibrium between the external and the internal reactant has been established, the circular precipitation area, originating from the site of the external reactant, is directly proportional to the amount of the antigen applied and inversely proportional to the concentration of the antibody in the gel. Standard antigen and standard antibody for use in this test are provided by the British National Institute for Biological Standards and Control (NIBSC), UK. It is to be noted that the SRID test has to be performed over at least two days.

An important drawback of the above methods as presently used in the field resides in the fact that evaluation of the primary virus yield of a given virus propagation culture according to the above methods can only be reliably performed after having separated the virus from the culture medium. However, purification of virus from large propagation cultures is both cost-intensive and time-consuming. Presently, it is not possible to evaluate the amount of virus in a given virus propagation culture within a period of several hours or less.

Thus, methods are needed which rapidly provide information as to the primary yield of a virus preparation, such as an influenza virus propagation culture. Preferably, the determination method should provide a result so rapidly that such result can be used as a basis for deciding whether or not the virus propagation culture is further processed and subjected to elaborate purification methods. The present invention solves this problem and provides additional benefits as well.

### Detailed Description of the Invention

The present invention provides a method for determining the concentration of viral particles and/or antigens in a virus-containing sample within a short period of time. The virus-containing sample can be a crude sample of a virus propagation culture, such as a culture of MDCK cells, and the method according to the invention is performed with an aliquot of said culture. Advantageously, examination of the aliquot can be performed without the necessity to subject the complete culture volume to downstream purification. Thus, the invention provides a method for determining the concentration of virus particles/virus antigens in a sample comprising the steps of
a) contacting a sample containing virus particles and/or virus antigens to an ion-exchange matrix under conditions that allow binding of said virus particles and/or virus antigens to said ion-exchange matrix, thereby separating said virus particles and/or virus antigens from other sample components;
b) eluting said bound virus particles and/or virus antigens from the matrix; and
c) detecting the virus particles and/or virus antigens;
wherein the detection signal is indicative for the concentration of said virus particles and/or virus antigens in the sample.

In course of the present invention, it has been found that there is a direct correlation between the amount of virus particles and/or virus antigens in a given sample and the amount of virus which can be bound to and eluted from an ion-exchange matrix, preferably a cation exchange matrix. This is surprising, since prior to the present invention such columns have never been contemplated for quantitative analysis of virus particles, such as influenza virus particles.

According to the method of the present invention, the virus particles and/or virus antigens in the sample are bound to an ion-exchange matrix in a first step. For example, any chromatography column which is equipped with an ion-exchange resin material, preferably a cation exchange material, capable of binding virus particles or proteins may be used. Such columns can readily be purchased from several manufacturers, such as SP Sepharose™ XL from GE Healthcare Life Sciences, Sartobind S from Sartorius, and the like. According to a preferred aspect of the invention, the column used in the method according to the invention comprises a cellulose matrix. According to a particularly preferred embodiment, the cellulose matrix has a gel exclusion limit of 2000 to 4000 Dalton, more preferably 3000 Dalton. The cellulose matrix preferably comprises activated groups. Preferred activated groups include, for example, sulfate ester or other weak cation exchange groups, such as Carboxymethyl (CM). According to a preferred embodiment, the cellulose matrix comprises sulfate ester as activated groups.

Usually, the sulfate esters are present on the cellulose matrix in a comparatively low concentration. For example, the matrix can comprise 500-900, preferably 700 µg sulfur per gram dry matrix material. A cellulose matrix which is particularly suitable for a method according to the invention can be purchased from Millipore GmbH, Eschborn, Germany, under the trademark Matrex® Cellufine™ Sulfate.

The sample containing the virus particles and/or virus antigens is contacted with the ion-exchange matrix under conditions that allow binding of said virus particles and/or virus antigens to said ion-exchange matrix. Preferably, such conditions are conditions of low ionic strength, i.e. binding of the virus particles and/or virus antigens in step a) is effected under low salt conditions. Preferably, contacting and binding is performed in a suitable buffer solution. Generally, the choice of the buffer is not critical, so that most buffer solutions commonly used for ion-exchange chromatography may be used. Thus, the buffer solution used for contacting the virus particles and/or virus antigens to the matrix can comprise NaCl, Na₂HPO₄, NaHPO₄, Na₃PO₄, Na₂SO₄, KCl, K₂HPO₄, KHPO₄, K₃PO₄, K₂SO₄, MgCl₂, NH₄Cl, and the like. The total salt concentration of the sample can be up to 0,1 mol/l, for example, 0,01 mol/l, or 0,05 mol/l. In case the material comprising the virus particles and/or virus antigens (for example an aliquot of a virus propagation culture) has a higher salt concentration, such material can be readily diluted with the above buffer solutions in order to provide a sample of low ionic strength which can be applied in the method of the invention. Furthermore, the material comprising the virus particles and/or virus antigens may be desalted before applying the sample to ion-exchange chromatography. Preferably, contacting of the virus particles and/or virus antigens in step a) is effected in the presence of a buffer solution comprising Na₂HPO₄. More preferably, the concentration of Na₂HPO₄ in the sample is between 0,01 and 0,1 mol/l. Most preferably, the concentration of Na₂HPO₄ in the sample is 0,05 mol/l. Contacting the sample with the matrix can be performed in a pH range of about 6,0 to 8,0, preferably 7,0 to 7,8. Most preferably, contacting is performed at a pH of 7,5. To get quantitative results, one has to ensure that the binding capacity of the respective matrix is by far higher than the amount of virus particles and/or virus antigens in the sample to be examined. If the concentration of virus particles and/or virus antigens is too high, dilution should be considered.

After the virus particles and/or virus antigens have been bound to the ion-exchange matrix, several washing steps may be performed in order to separate contaminating components of the sample, for example cellular debris originating from host cells used for virus propagation or medium components, from the virus particles and/or virus antigens. Washing can be performed according to common methods used in the field of chromatography using well known buffers of low ionic strength. For an overview of numerous chromatography methods and techniques, reference is made to "Ion Exchange Chromatography, Principles and Methods", Amersham Pharmacia Edition AA (1998). Preferably, the same buffer which has been used for contacting the virus particles and/or virus antigens to the ion-exchange matrix is used for washing. According to one aspect of the invention, it is preferred to use a sodium phosphate buffer, such as Na₂HPO₄, having a concentration of 10 to 100 mmol/l for washing. For example, the buffer may have a concentration of 20, 40, 60, 80, or 100 mmol/l. Preferably, the Na₂HPO₄buffer has a concentration of 50 mmol/l. As will be appreciated by those skilled in the art, the washing buffer may contain several other components, such as further buffering agents like TRIS (Tris(hydroxymethyl)-aminomethan), MOPS (3-(N-morpholino)-Propansulfonsaure) or HEPES (N-(2-Hydroxyethyl)-piperazin-N'-ethansulfonsaure) or detergents like Triton-X 100, sodium dodecyl sulfate, polysorbate, polyoxyethylene sorbitol esters such as polysorbate 80 (Tween-80) or polysorbate 20 (Tween-20), polyoxypropylene-polyoxyethylene esters such as poloxamer 188, polyoxyethylene alcohols such as BRIJ 35, and the like.

Subsequently, the virus particles and/or virus antigens are eluted from the column by use of a buffer containing substances which interfere with the interaction of the virus particles and/or virus antigens with the ion-exchange matrix. Such buffers may comprise the same salts as the buffers used for binding the virus particles and/or virus antigens to the column (see above), however in higher concentrations. Thus, elution of the virus particles and/or virus antigens in step c) is effected under high salt conditions. The concentration of the buffer used for elution depends on the nature of the buffer. For example, the salt concentration of the elution buffer may range from about 0,8 to 5 mol/l. Specifically, the salt concentration of the elution buffer may be 1, 1,2, 1,5, 1,8, 2, 2,2, 2,5, 2,8, 3, 3,5 4, or 4,5 mol/l. Preferably, eluting the virus particles and/or virus antigens in step c) is effected in the presence of a NaCl buffer. The NaCl buffer preferably has a concentration of between 1,0 and 2,0 mol/l, more preferably 1,2 mol/l. Alternatively, an NH₄SO₄ buffer having a concentration of between 1,0 and 2,0 mol/l can be used for elution. Elution can also be performed in two or more steps using buffers of different concentrations. According to a preferred embodiment of the invention, the elution is performed in two steps, wherein a first elution is performed by use of a sodium chloride buffer of 1,2 mol/l, and subsequently, a second elution is performed by use of a sodium chloride buffer of 3 mol/l. It will be understood by those skilled in the art that elution can also be performed by a linear gradient with increasing salt concentrations.

According to the invention, the concentration of both complete virus particles and/or specific parts thereof, such as surface proteins or other protein components (referred to as virus antigens herein), can be determined. As shown in the examples, the method of the invention is particularly suitable to determine the concentration of complete virus particles, such as influenza virus particles, in a sample. Thus, the method provides for determining the yield of a virus propagation culture, such as a MDCK cell culture which has been infected with a virus, such as an influenza virus. Alternatively, if only certain protein components of a virus are produced for subsequent use in vaccines, the present method is also useful for determining their concentration. Moreover, both the concentration of complete virus particles and virus antigens can be determined in parallel, provided that they elute with different retention times.

When eluting the virus particles and/or virus antigens, the virus-related substances eluting from the column are detected by suitable means. For example, absorbance by the eluting substances may be measured at a specific wave length. The choice of the wave length depends on the nature of the virus to be detected. For example, virus particles such as influenza virus particles can be detected in a range of 210 to 350 nm, for example 280 nm. According to a preferred aspect of the invention, the eluting substances are detected by absorbance measurement at a wave length of 280 nm. For this purpose, common UV detection means which are well known in the field of chromatography may be used.

The virus particles and/or virus antigens to be determined according to the method of the present invention can originate from different types of viruses, for example, from viruses of the families orthomyxoviridae, adenoviridae, herpesviridae, arenaviridae, flaviridae, retroviridae, hepadnaviridae, papovaviridae, parvoviridae, poxviridae, and the like. Preferred viruses are HCV, measles virus, HAV, HBV, vaccinia virus, lassa virus, HSV-1, HSV-2, yellow fever virus, rubella virus, polio virus, HIV and influenza virus. Preferably, the virus particles and/or virus antigens originate from an influenza virus. The influenza virus may be selected, for example, from the strains A/New Caledonia H1N1, B/Jingsu, A/Wyoming H3N2, A7New York H3N2, B/Malaysia and other strains. The sample, which comprises the virus particles and/or virus antigens preferably can be an aliquot of a virus propagation culture. As used herein "virus propagation culture" means any culture which serves the purpose of propagating viruses or virus antigens. The term in particular includes any kind of cell or tissue culture, in which the cells or tissues, for example vertebrate cells, are used as a host for the replication of the virus. The aliquot may be taken from the virus propagation culture at the end of the culturing process. The method according to the invention allows to determine the concentration of virus particles and/or virus antigens in the sample. The concentration can readily be extrapolated to the complete culture volume. According to the invention, the sample comprising the virus particles and/or virus antigens may be derived from an egg culture or from a cell culture, such as a mammalian cell culture. According to a preferred embodiment, the sample is derived from an MDCK, MDBK, VERO, CV-1, MRC-5, WI-38, or an LLC-MK2 cell culture. According to a even more preferred embodiment, the sample is derived from an MDCK cell culture.

The results obtained from the determination method according to the invention can be used as a basis for deciding as to whether a complete virus propagation culture should be further processed and/or purified. Since downstream processing and/or purification of a complete virus propagation culture is extremely cost and labor intensive, the method of the invention provides for a more economic and cost-saving production approach. Cultures which exhibit only a low virus/antigen content at the end of the culturing phase according can be discarded and do not have to be subjected to downstream processing and/or purification methods. For example, an acceptable yield of an influenza virus propagation culture can be about 10-20 µg/ml culture. However, the yield to be expected will differ, dependent on the virus type and the culture method. Alternatively, the determination result can also be the basis for deciding as to whether the virus propagation culture is to be further incubated and/or the culture conditions are to be optimized. It will be understood that in the latter case the aliquot of the virus propagation culture must be obtained under sterile condition.

According to a preferred embodiment of the invention, the method for determining the concentration of virus particles and/or virus antigens in a sample further comprises the step of comparing the detection signal to at least one reference value obtained from detecting a sample containing said virus particles and/or virus antigens in a known concentration (reference sample) in order to determine the concentration of virus particles and/or virus antigens in the sample. In other words, the specific equipment used for the method of the invention is calibrated prior to applying the sample with the unknown concentration of virus particles and/or virus antigens. The detection signal obtained with the reference sample can be correlated to a specific concentration of virus particles and/or virus antigens. Preferably, the detection signal is compared to more than one reference value. For example, a calibration curve may be prepared by determining the detection signals of several samples of different known concentrations. The samples may contain between 5 and 50 µg virus particles and/or virus antigens per ml of the reference sample. Typically, reference samples with increasing concentration of virus particles and/or virus antigens are used. For example, samples containing 5 µg, 10 µg, 15 µg, 20 µg, 25 µg, 30 µg, 35 µg, 40 µg, 45 µg, and 50 µg virus particles and/or virus antigens per ml may be used to generate a calibration curve. The detection signal obtained from a sample of unknown concentration of virus particles and/or virus antigens can then be compared to the calibration curve in order to assess the concentration of virus particles and/or virus antigens. Preferably, reference values are established for each virus type. It may also be necessary in some cases to establish reference values for different strains of a given virus type (such as in the case of influenza). For example, reference values can be established for the influenza strains A/New Caledonia H1N1, B/Jingsu, A/Wyoming H3N2, A7New York H3N2, B/Malaysia. In several cases it may also be found out that reference values established on the basis of one strain may allow for measuring and assessing samples containing virus particles and/or virus antigens of another strain. Strain specific correlation factors may be established which allow for using such reference values. If the same equipment is used with the same experimental conditions (i.e. the same detector settings, flow rate etc.), it is also possible to use the detector signal (as expressed, for example, by the specific area of a measured peak) as an indicator for the concentration of virus particles and/or virus antigens. In the examples described below, the peak area is directly correlated to the concentration of virus particles and/or virus antigens as measured by standard methods (SRD and HA titer, respectively).

When culturing influenza virus in cell cultures, infection of the host cells is dependent on several factors, such as the addition of proteases like trypsin to the infection medium. The proteases extracellularly cleave the precursor protein of hemagglutinin into active hemagglutinin which is involved in the process of cell infection. However, protease addition does often not lead to reproducible results in every batch. Moreover, since protease addition requires opening the culturing vessel at least once, there is a high risk of contaminating the virus propagation culture. Such contamination, however, would render the virus product obtained from the cell culture unsuitable for subsequent preparation of vaccines.

According to another preferred aspect, the method according to the invention allows for assessing the virus propagation culture in terms of quality. For example, if microorganisms other than the virus to be propagated have contaminated the culture, such contamination would be detectable by the method of the invention, either by a low yield of virus or by occurrence of further peaks in the chromatographic analysis. Such contaminated cultures would be discarded and not subjected to downstream purification. The method according to the invention preferably is for determining the concentration of complete virus particles which are present in a sample. However, it is also possible to determine the concentration of other virus antigens, such as distinct surface proteins. It has been found in course of the invention that the concentration of complete influenza virus particles grown in MDCK cell cultures is low, if the concentration of free (i.e. not virus-associated) influenza virus surface proteins is high. Thus, the concentration of such surface proteins can also be used as an indication as to the yield of viral particles in a sample.

Finally, the invention relates to the use of a ion-exchange matrix, preferably a cation exchange matrix, for the determination of the concentration of virus particles and/or virus antigens in a sample. Preferably, the ion-exchange matrix is a cation-exchange matrix.

### Brief description of the figures:

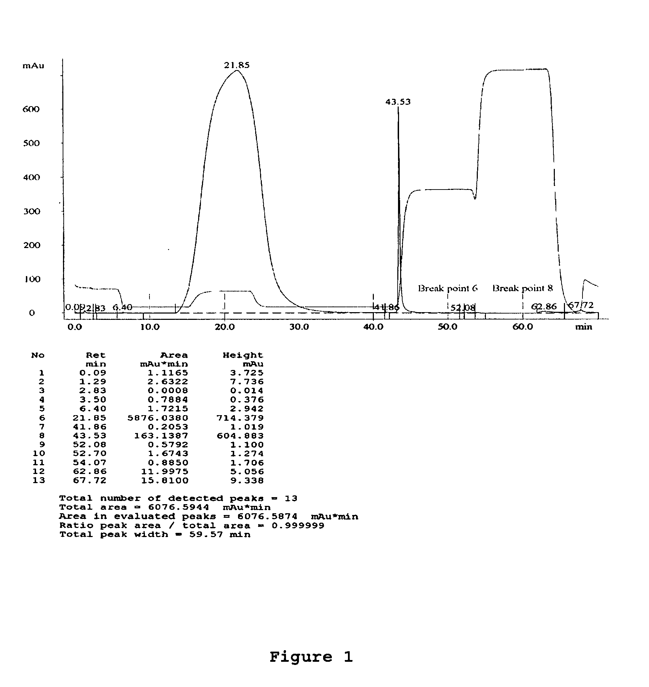

Figure 1 shows a chromatogram obtained by running a sample containing influenza virus particles on a Matrex® Cellufine™ sulfate column using the ÄKTAprime chromatographic system. 50 ml fermenter harvest of an influenzs virus, strain A/Wellington, was added to the column. The peak at 43.53 minutes represents the influenza virus particle fraction. As shown in SDS-PAGE analysis, the virus particle fraction exhibited a purity of more than 80% (data not shown).

### EXAMPLES

It has to be understood that the following examples merely illustrate the present invention and are not intended to limit the invention as defined in the attached claims.

The following equipment was used:
**1. Equipment A**
   ➢ Chromatography system: ÄKTAprime (GE healthcare), 50 ml sample loop, online detection of UV absorbance at 280nm, online detection of conductivity
   ➢ Column: HR 16/10 (GE healthcare), 20ml Matrex® Cellufine™ sulfate (Millipore)
   ➢ Washing buffer: 50 mM Na₂HPO₄, adjusted with 1M NaOH to pH 7,5
   ➢ Elution buffer 1: 1,2 M NaCl, 50 mM Na₂HPO₄, adjusted with 1 NaOH to pH 7,5
   ➢ Elution buffer 2: 3,0 M NaCl, 50 mM Na₂HPO₄, adjusted with 1 NaOH to pH 7,5
   ➢ Cleaning buffer: 0,1 NaOH
   ➢ Storage buffer: Washing buffer + 20% Ethanol
   ➢ Programming: Flowrate 5ml
      - 10 min (50ml): column equilibration
      - 10 min (50ml): sample injection
      - 20 min (100ml):column washing with washing buffer
      - 10 min (50ml): elution with elution buffer 1
      - 10 min (50ml): elution with elution buffer 2
      - 10 min (50ml): Cleaning with cleaning buffer
**2. Equipment B**
   ➢ Chromatography system: ÄKTAbasic (GE healthcare), 2 ml sample loop, online detection of UV absorbance at 280nm and 350nm
   ➢ Column: Tricorn 10/20 (GE healthcare), 2 ml Matrex® Cellufine™ sulfate (Millipore)
   ➢ Washing buffer: 50 mM Na₂HPO₄, adjusted with 1M NaOH to pH 7, 5
   ➢ Elutionbuffer : 3,0 M NaCl, 50 mM Na₂HPO₄, adjusted with 1 M NaOH to pH 7,5
   ➢ Cleaning buffer: 0,1 M NaOH
   ➢ Storage buffer: Washing buffer + 20% Ethanol
   ➢ Programming: Flowrate 5ml
      - 2 min (10ml): sample injection and column washing with washing buffer
      - 2 min (10ml): elution with 30% elution buffer, 80% washing buffer
      - 2 min (10ml): elution with 100% elution buffer
      - 2 min (10ml): Cleaning with 100% cleaning buffer
      - 2 min (10ml): column equilibration with washing buffer

### EXAMPLE 1

### Chromatographic analysis of a influenza virus sample

A sample derived from a fermenter harvest of a MDCK (Madin-Darby canine kidney) cell culture infected with influenza strain A/Wellington was filtrated using a 0,45 µm filter before applying to the chromatography column. A flowrate of 5 ml/min was used. The column was equilibrated for 10 min with washing buffer. Then 50 ml of the sample were injected. The column was washed with 100 ml washing buffer. Elution with 50 ml elution buffer 1 was performed to elute the complete viruses and a further elution step with 50 ml elution buffer 2 was performed to elute viral surface proteins which are not associated with complete virus particles. The latter proteins might be the product of incomplete virus production. Finally the column was rinsed with 50 ml cleaning buffer.

Figure 1 shows the absorption spectrum that was obtained for the elution. The peak at 43.53 min represents the virus particle fraction. The peak area reveals that the virus propagation culture is sufficient for further processing of the sample to produce e.g. a vaccine. It was also found out that viral surface proteins which are not associated with complete virus particles can be used as a further indication of the yield of virus particles from the virus propagation culture. The higher the concentration of these unbound viral proteins, the lower the concentration of virus particles is. If the production of complete virus particles is aimed at, the area of the peak at 43.53 min should be large, and no peak (or a peak having a small area) should be obtained when elution with 3 M NaCl is performed) .

### EXAMPLE 2

### Correlation of results from chromatographic analysis and SRD analysis of an influenza virus sample

Different influenza virus strain were propagated in MDCK cultures according to known methods. At the end of the culturing phase, the concentration of virus particles and/or virus antigens in a 2 ml sample was determined by analytical CS-chromatography (Matrex® Cellufine™ sulfate; Millipore) after filtration using a 0,45 µm filter for removing cellular debris. The established HA titration method was performed in parallel using an equal sample. The confidence limit of the SRD determination is +20%. The results obtained are listed in Table 1.

**Table 1: results of determination of the concentration of virus particles by SRD and analytical CS-chromatography**

| Lot | strain | SRD value in the primary yield | CS Peak area of the virus peak |
|---|---|---|---|
| OOCL110503 | A/New Caledonia H1N1 | 10 µg/ml | 282 |
| OOCL110504 | A/New Caledonia H1N1 | 6 µg/ml | 196 |
| OOCC110412 | B/Jiangsu | 10 µg/ml | 92 |
| OOCC110413 | B/Jiangsu | 10 µg/ml | 94 |
| OOCB110501 | A/Wyoming H3N2 | 14 µg/ml | 172 |
| OOCY110506 | A/New York H3N2 | 8 µg/ml | 67 |
| OOCY110507 | A/New York H3N2 | 10 µg/ml | 74 |
| OOCY110508 | A/New York H3N2 | 14 µg/ml | 173 |

### EXAMPLE 3

### Correlation of the results from chromatographic analysis and HA titration analysis of an influenza virus sample

Different influenza virus strain were propagated in MDCK cultures according to known methods. At the end of the culturing phase, the concentration of virus particles and/or virus antigens in a 2 ml sample was determined by analytical CS-chromatography (Matrex® Cellufine™ sulfate; Millipore) after filtration using a 0,45 µm filter for removing cellular debris. The established HA titration method was performed in parallel using an equal sample. The confidence limit of the SRD determination is +20%. The results obtained are listed in Table 2.

**Table 2: results of determination of the concentration of virus particles by HA titration and analytical CS-chromatography**

| Lot | strain | HA titer | CS Peak area of the virus peak |
|---|---|---|---|
| 201005RK-1/24 | B/Malaysia | 32 | 26 |
| 201005RK-3/24 | B/Malaysia | 32 | 19 |
| 201005RK-4/24 | B/Malaysia | 256 | 41 |
| 201005RK-1/48 | B/Malaysia | 512 | 93 |
| 201005RK-4/48 | B/Malaysia | 1024 | 198 |
| 100505EV-4/24 | A/New York H3N2 | 128 | 40 |
| 100505EV-3/48 | A/New York H3N2 | 256 | 44 |
| 100505EV-3/72 | A/New York H3N2 | 512 | 91 |
| 100505EV-4/72 | A/New York H3N2 | 1024 | 119 |

It can be inferred from the tables that the peak area of the respective virus peak correlates strongly with the concentration of virus particles and/or virus antigens in the sample as determined by common methods. The correlation of the peak area to the concentration values as determined by SRD and HA-titer differs between all tested influenza virus strains. The specific absorption at 280 nm seems to differ between the tested virus strains. It could be assumed that a strain specific correlation factor could compensate this observation.

As the analytical CS-chromatography can be performed in less than one hour, this method significantly enhances the time-consuming optimizing of the infection process. In contrast to the 2-3 day lasting SRD method, the analytical CS-chromatography could be used as an in-process-control e.g. to decide the optimal harvesting time of a production scale fermentor.

## Claims

1. Method for determining the concentration of virus particles and/or virus antigens in a sample comprising the steps of
a) applying a sample containing virus particles and/or virus antigens to an ion-exchange matrix under conditions that allow binding of said virus particles and/or virus antigens to said ion-exchange matrix, thereby separating said virus particles and/or virus antigens from other sample components;
b) eluting said bound virus particles and/or virus antigens from the matrix; and
c) detecting the virus particles and/or virus antigens;
wherein the detection signal is indicative for the concentration of said virus particles and/or virus antigens in the sample.

2. Method according to claim 1, wherein the method further comprises the step of comparing the detection signal to at least one reference value obtained from detecting a sample containing said virus particles and/or virus antigens in a known concentration in order to determine the concentration of virus particles and/or virus antigens in the sample.

3. Method according to claim 2, wherein the detection signal is compared to more than one reference value.

4. Method according to any of claims 1 to 3, wherein said ion-exchange matrix is a cation-exchange matrix.

5. Method according to claim 4, wherein said cation-exchange matrix comprises a cellulose matrix.

6. Method according to claim 5, wherein said cellulose matrix has a gel exclusion limit of 2000 to 4000 Dalton.

7. Method according to any of claims 5 or 6, wherein said cellulose matrix comprises sulfate ester as activated groups.

8. Method according to any of claims 1 to 7, wherein the method is for determining the concentration of virus particles.

9. Method according to any of claims 1 to 8, wherein the virus is an influenza virus.

10. Method according to claim 9, wherein the virus is influenza virus selected from the strains A/New Caledonia H1N1, B/Jingsu, A/Wyoming H3N2, A7New York H3N2, B/Malaysia.

11. Method according to any of claims 1 to 10, wherein said sample is an aliquot of a virus propagation culture.

12. Method according to claim 11, wherein said sample is the derived from an egg culture.

13. Method according to claim 11, wherein said sample is derived from a cell culture.

14. Method according to claim 13, wherein said sample is derived from an MDCK cell culture.

15. Method according to any of claims 1 to 14, wherein binding of the virus particles and/or virus antigens in step a) is effected under low salt conditions.

16. Method according to claim 15, wherein contacting of the virus particles and/or virus antigens in step a) is effected in the presence of a buffer comprising Na₂HPO₄.

17. Method according to claim 16, wherein the concentration of Na₂HPO₄ in said buffer is between 0,01 and 0,1 mol/l.

18. Method according to any of claims 1 to 17, wherein eluting the virus particles and/or virus antigens in step c) is effected under high salt conditions.

19. Method according to claim 18, wherein eluting the virus particles and/or virus antigens in step c) is effected in the presence of a NaCl buffer.

20. Method according to claim 19, wherein the concentration of NaCl in said buffer is between 1,0 and 2,0 mol/l.

21. Method according to any of claims 1 to 20, wherein detection in step c) is effected by measuring UV absorbance of the elution fractions.

22. Method according to claim 21, wherein UV adsorption is measured at a wave length of 280 nm.

23. Use of a ion-exchange matrix for the determination of the concentration of virus particles and/or virus antigens in a sample.

24. Use according to claim 23, wherein said ion-exchange matrix is a cation-exchange matrix.
